# EUROPEAN PATENT SPECIFICATION

(11) **EP 3 202 332 B1**
(45) Date of publication and mention of the grant of the patent: **01.07.2020**
(21) Application number: 17154588.2
(22) Date of filing: 03.02.2017
(51) Int. Cl.: A61B 10/00

(54) **A CONTAINER FOR COLLECTING FAECAL SPECIMENS**
BEHÄLTER ZUR ENTNAHME VON FÄKALPROBEN
RÉCIPIENT DE COLLECTE D'ÉCHANTILLONS DE MATIÈRES FÉCALES

(30) Priority: 05.02.2016 IT UB20160397
(43) Date of publication of application: 09.08.2017
(73) Proprietor: THD S.p.A., 42015 Correggio, Reggio Emilia (IT)
(72) Inventor: Bastia, Filippo, 41019 Soliera (Modena) (IT)
(74) Representative: Paparo, Aldo

(56) References cited:
- EP-A1- 2 325 619
- WO-A1-2012/150713
- US-A- 5 624 554
- US-A1- 2006 115 385

## Description

The present invention relates to a container for collecting faecal specimens, as defined in claim 1.

In particular, the present invention relates to the field of containers for collecting of specimens intended for analyses for detecting the presence of indicators of pathologies. Such indicators can be, for example, helicobacter pylori or HVC RNA for the diagnosis of hepatitis C or others.

The device can likewise be used to detect any genetic profile known to be involved in various viral, bacterial and neoplastic pathologies.

In the state of the art are known different type of containers as the ones describes in documents US5624554, US2006/115385, WO2012/150713 and EP2325619.

The containers of the above-mentioned type comprise an openable and closable outer casing provided, generally in the cap, with a wand shaped in such a manner as to facilitate the withdrawal of the correct amount of specimen. Inside the casing there is already present an extraction solution having the purpose of dissolving the collected faeces and preparing them for the subsequent analyses. There are two known methods for analyzing the extraction solution thus obtained. In accordance with a first method, it is the user himself who shakes the container and withdraws a certain amount of extraction solution to be qualitatively analyzed, for example by means of reagent paper strips. This first method is scarcely reliable, due both to the fact that it leaves the step of analysis up to the user and the fact that it uses qualitative reading instruments.

In accordance with a second method, the closed container is sent off to a test centre in which a qualified operator extracts the extraction solution and analyses it quantitatively.

Unfortunately, this second method, too, is not without limitations due to the fact that the containers can undergo damage during transport.

In particular, there are some known types of containers in which a breakage zone is specifically provided to enable access to the extraction solution.

In addition to the above, the known containers have intrinsic disadvantages mainly determined by the fact that the withdrawn extraction solution can be contaminated by particles of the specimen that have remained undissolved.

In this context, the technical task at the basis of the present invention is to propose a container for collecting faecal specimens which overcomes the drawbacks of the aforementioned prior art.

In particular, it is an object of the present invention to provide a container for collecting faecal specimens capable of being safely transported for the subsequent analyses to be carried out at a qualified centre.

A further object of the present invention is to propose a container for collecting faecal specimens that ensures an optimal quality of the withdrawn extraction solution, such as to guarantee a high reliability of the results.

The stated technical task and specified objects are substantially achieved by a container for collecting faecal specimens comprising the technical features disclosed in one or more of the appended claims. The dependent claims correspond to different embodiments of the invention.

In particular, the present invention relates to a container for collecting faecal specimens comprising an extraction portion suitable for containing an extraction solution and a closure portion for closing the container. A connection means is interposed between the extraction portion and the closure portion and configured to define a closed configuration of the container. A shaped wand is suitable for collecting the specimens and suitable for being axially inserted into the extraction portion when the container is in the closed configuration. The container comprises at least one filtering element which, at least in the closed configuration of the container, divides a solubilization chamber from a sampling chamber. The filtering element is suitable for enabling a flow of the extraction solution from the solubilization chamber to the sampling chamber.

By providing two chambers separate from the filtering element, one obtains a compact container that can be easily and safely transported and is capable of ensuring an optimal reliability of the results.

Preferably, the extraction portion comprises at least partially the solubilization chamber.

Preferably, in the closed configuration, the shaped wand is suitable for being axially inserted into the solubilization chamber. The sampling chamber is disposed on the opposite side of the solubilization chamber relative to the shaped wand.

Preferably, there is provided a passage means configured to enable the flow of the extraction solution from the solubilization chamber to the sampling chamber.

Preferably, the passage means comprises at least one opening fashioned in the shaped wand and configured to enable the flow of the extraction solution from the solubilization chamber to the sampling chamber.

Preferably, the shaped wand comprises a plurality of projections extending in a radial direction. The space between two consecutive projections defines an opening configured to enable the flow of the extraction solution from the solubilization chamber to the sampling chamber.

Preferably, the shaped wand comprises at least one perimeter ring for joining the projections. The perimeter ring is suitable for abutting against a free edge of the extraction portion.

Preferably, the projections extend axially along a discrete portion of the shaped wand, thus forming fins.

Preferably, the projections extend axially along a discrete portion of the shaped wand equal to 1/3 of the axial length of the shaped wand.

Preferably, the shaped wand is associated with said closure portion.

Preferably, the filtering element is associated with said closure portion.

Preferably, the filtering element is associated with the shaped wand. Preferably, the filtering element is packed between the shaped wand and the closure portion.

Preferably, the closure portion comprises at least partially the sampling chamber.

Preferably, the sampling chamber, for example disposed in the closure portion, comprises at least one sampling point closed off by means of at least one pierceable layer.

Preferably, the extraction portion comprises a penetrable or removable element disposed so as to close off the extraction portion in an open configuration of the container.

Preferably, said penetrable or removable element is defined by a membrane.

The shaped wand comprises a tip configured to tear the penetrable element during the passage from the open configuration to the closed configuration of the container.

Preferably, the container has an at least partially tubular conformation along an axis of extension. At least in the closed configuration, the sampling chamber and the solubilization chamber are disposed in opposite portions of the container along said axis of extension.

Preferably, there is provided a supporting base configured to be associated with a machine for treating the extraction solution, for example a centrifuge. The supporting base is preferably associated with the extraction portion.

Preferably, the supporting base has a cup-like conformation.

Preferably, the filtering element comprises a polypropylene membrane.

Additional features and advantages of the present invention will become more apparent from the approximate, and hence non-limiting, description of a preferred but non-exclusive embodiment of a container for collecting faecal specimens, as illustrated in the appended drawings, in which:
- figure 1 is a schematic front view of a container for collecting faecal specimens according to the present invention;
- figure 2 is a sectional view according to the dashed line II-II of the container for collecting faecal specimens of figure 1;
- figure 3 is a sectional view according to the dashed line II-II of a part of the container for collecting faecal specimens of figure 1, prior to closure;
- figure 4 is a sectional view according to the dashed line II-II of a part of the container for collecting faecal specimens of figure 1, prior to closure;
- figure 5 is a perspective view in separate parts of part of the container for collecting faecal specimens of figure 4;
- figure 6 illustrates the container of figure 2 in a different operating condition;
- figure 7 and figure 8 respectively illustrate two schematic sectional views of possible embodiments of the container for collecting faecal specimens according to the present invention.

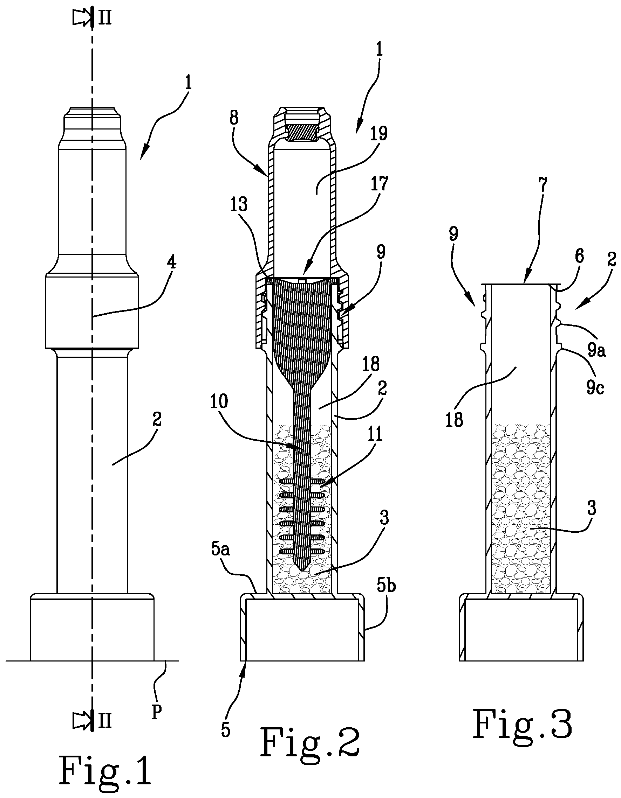

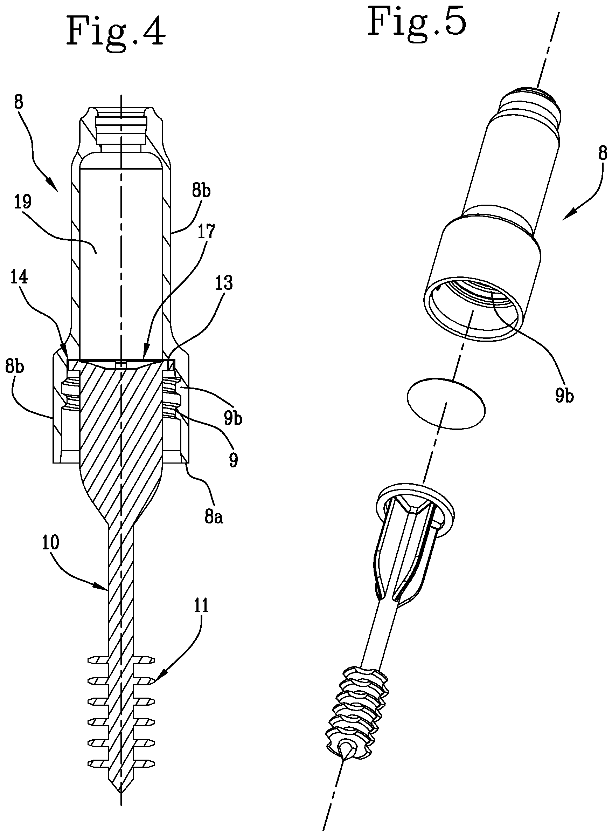

With reference to figures 1-6, 1 denotes in its entirety a container for collecting faecal specimens, hereinafter simply referred to as the container 1. With reference to figures 1-3, the container 1 is illustrated in a vertical position, resting on a horizontal rest surface P.

The container 1 comprises an extraction portion 2 suitable for containing an extraction solution 3. Extraction solution means a solution suitable for dissolving at least partially the faeces collected and preparing them for the subsequent analyses. For example, the extraction solution can be made up of an aqueous saline solution containing sodium chloride, sodium phosphate and, in some formulations, potassium chloride (PBS solution). In accordance with a possible embodiment, the extraction portion 2 has an at least partially tubular conformation along an axis of extension 4. In other words the extraction portion 2 has an axial dimension that is greater than the transversal dimensions and a cylindrical conformation with a circular base.

Preferably, the container 1 comprises a supporting base 5 configured to be associated with a machine for treating the extraction solution 3, for example a centrifuge. In accordance with the non-limiting example illustrated in the appended figures, the supporting base 5 is preferably associated with the extraction portion 2. In particular, the supporting base 5 is made in one piece with the extraction portion 2.

As may be seen in figures 1-3 the supporting base 5, in one embodiment, can have a cup-like conformation with the opening facing downward. In other words, the supporting base 5 comprises a widened plate 5a that extends transversely relative to the axis of extension 4 and cylindrical walls 5b that extend from the widened plate in a direction parallel to the axis of extension 4.

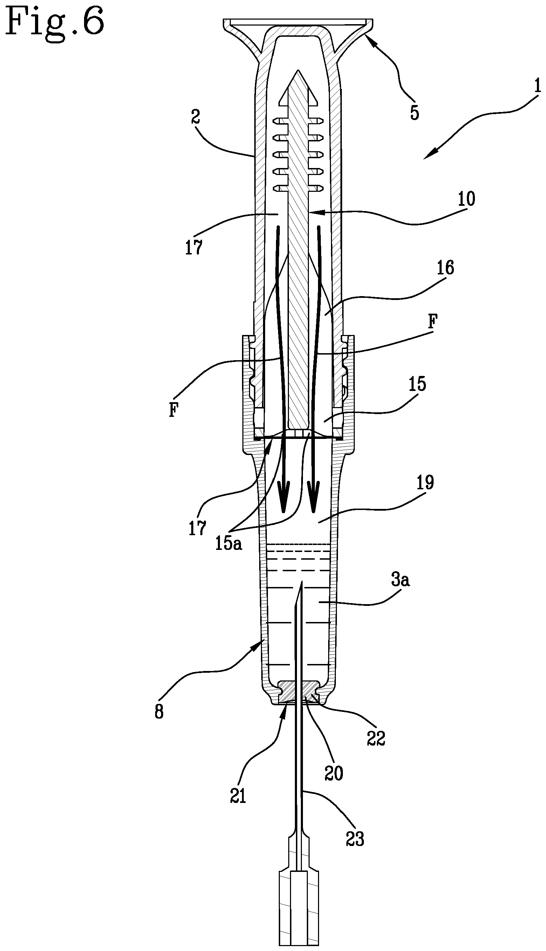

In another variant illustrated in figure 6, the supporting base 5 has a foot 5a, extending upward from which there is a tapered cylindrical wall 5b placed in connection with the extraction portion 2.

In both variants, the supporting base 5 is disposed so as to close off the extraction portion.

With reference to the appended figures, the supporting base 5 is disposed at a lower end of the container 1, considering a positioning thereof on the horizontal rest surface P.

The extraction portion 2 comprises a free edge 6 defining a mouth for accessing the inside of the extraction portion itself. In particular, the free edge 6 defines the only mouth for accessing the inside of the extraction portion. Preferably, the free edge 6 is disposed at one end of the extraction portion, in particular at an end opposite the supporting base 5, if present. For example the free edge 6 is disposed at an upper end of the container 1, considering a positioning thereof on the horizontal rest surface P.

In a tubular conformation of the extraction portion 2, the free edge 6 is defined by one end of the tube.

In accordance with a possible embodiment, the extraction portion 2 comprises a penetrable or removable element 7, disposed so as to close off the extraction portion, in particular the free edge 6, in an open configuration of the container 1 (figure 3).

Hereinafter in the present description, the term membrane shall equally describe the penetrable element 7, i.e. which may be penetrated by a pointed member, or the removable element 7, i.e. which may be peeled off of the seat accommodating it.

In an open configuration of the container 1, prior to the use thereof, the extraction solution 3 is disposed inside the extraction portion 2 and isolata by means of the penetrable membrane 7.

The container 1 further comprises a closure portion 8 suitable for closing the container 1. Preferably, the closure portion 8 has an at least partially tubular conformation along an axis of extension, coinciding, in particular, with the axis of extension 4 of the extraction portion 2. In a closed configuration of the container 1, for example illustrated in figures 1 and 2, the container 1 as a whole has an at least partially tubular conformation along the axis of extension 4.

The closure portion 8 comprises a free edge 8a which, in a closed configuration of the container 1, is facing towards the extraction portion. The free edge 8a defines a mouth for accessing the inside of the closure portion 8.

9 indicates a connection means interposed between the extraction portion 2 and the closure portion 8. The closing means 9 is configured to define the closed configuration of the container 1.

In accordance with a possible embodiment, the closing means 9 is of the threaded type and comprises an outer thread 9a, preferably associated with the extraction portion 2, and an inner thread 9b, preferably associated with the closure portion 8.

In particular, the closure portion 8 comprises a coupling collar 8b comprising the inner thread 9b and conformed to as be disposed externally to the extraction portion 2.Preferably, the closure portion 8 is made in one piece. Said closure portion is defined by a single monolithic element. In other words, said closure portion 8 is without any detachable or reversibly removable parts.

At the coupling collar 8b of the closure portion 8 there is a seat. Said seat is preferably defined by a through hole establishing a communication between the inside of the closure portion 8 and the outside thereof.

In accordance with a possible embodiment, the closing means 9 can comprise an abutting rib 9c, preferably associated with the extraction portion 2.

Said abutting rib 9c also acts as a seal for generating a tight closure of the closure portion 8 on the extraction portion 2.

10 indicates a shaped wand suitable for collecting specimens. The shaped wand 10 is suitable for being axially inserted into the extraction portion 2 when the container 1 is in the closed configuration, as is for example illustrated in figure 2.

The shaped wand 10 extends along an axis of extension thereof which, in the closed configuration of the container 1, substantially coincides with the axis of extension 4.

11 indicates a collection portion of the shaped wand 10, preferably made in comb-like fashion and disposed at one end of the shaped wand itself. In accordance with a possible embodiment, the shaped wand 10 comprises a tip 12 configured to tear the penetrable membrane 7 during the passage from the open configuration to the closed configuration of the container 1. Preferably, the shaped wand 10 is associated with the closure portion 8, for example by means of a snap fit, welding or connections of another nature. In particular, the shaped wand 10 comprises a perimeter ring 13 suitable for being housed in a seat 14 of the closure portion 8, for example disposed inside the coupling collar 8b. In other words, the shaped wand 10 is associated with the closure portion 8 in such a way as to be partially inserted in it.

In accordance with a possible embodiment, not illustrated, the shaped wand 10 is associated with the closure portion 8, being made in one piece with the closure portion itself.

In accordance with a possible embodiment, for example illustrated in the appended figures, the perimeter ring 13 is suitable for abutting against the free edge 6 of the extraction portion 2.

In accordance with a possible embodiment, the shaped wand 10 comprises a plurality of projections 15 extending in a radial direction. Preferably, the peripheral ring 13 is a ring for joining the projections 15. In the example illustrated in the figures, there are four projections evenly distributed around the axis of extension 4.

Preferably, the projections 15 extend axially along a discrete portion of the shaped wand 10, thus forming fins 16. In particular, the fins 16 extend for a portion equal to about 1/3 of the axial length of the shaped wand 10.

The fins 16 have a radial dimension that is greater than the radial dimension of the remaining part of the shaped wand 10. In particular, the fins 16 are suitable for centring the shaped wand 10 inside the extraction portion 2.

Advantageously, the container 1 comprises at least one filtering element 17 which, at least in the closed configuration of the container, divides a solubilization chamber 18 from a sampling chamber 19.

In particular, the filtering element 17 is suitable for enabling a flow F of the extraction solution from the solubilization chamber 18 to the sampling chamber 19, where the filtered extraction solution 3a is collected.

Preferably, the filtering element 17 comprises a polypropylene membrane. Said filtering element 17 has a membrane with a pore size ranging preferably from 90 to 180 microns (µm), even more preferably the pores are about 130 µm.

According to a possible embodiment, for example illustrated in the figures, the extraction portion 2 comprises at least partially the solubilization chamber 18 and the closure portion 8 comprises at least partially the sampling chamber 19.

In particular, in the closed configuration, the sampling chamber 19 and the solubilization chamber 18 are disposed in opposite portions of the container 1 along the axis of extension 4. With reference to the closed configuration, the shaped wand 10 is suitable for being axially inserted into the solubilization chamber 18, as illustrated in figure 2, and the sampling chamber 19 is disposed on the opposite side of the solubilization chamber 18 relative to the shaped wand 10.

In accordance with a possible embodiment, the filtering element 17 is associated with the closure portion 8. Preferably, the filtering element 17 is packed between the shaped wand 10 and the closure portion 8. According to a possible embodiment, for example illustrated in the appended drawings, the filtering element 17 is suitable for being housed in the seat 14 of the closure portion 8, between the peripheral ring 13 and the closure portion itself. In other words, according to a possible example, the filtering element is disposed at least partially in contact with the shaped wand 10. In a possible alternative embodiment, the filtering element is disposed inside the closure portion 8.

In a further alternative embodiment, the filtering element 17 is entirely associated with the shaped wand 10.

In general terms, the container 1 comprises a passage means configured to enable the flow F of the extraction solution from the solubilization chamber 18 to the sampling chamber 19. In particular, the passage means can be defined by the space between two consecutive projections 15 of the shaped wand 10, wherein said space defines an opening 15a configured to enable the flow F of the extraction solution from the solubilization chamber 18 to the sampling chamber 19 (figure 6). In general, the passage means can be made up of at least one opening fashioned in the shaped wand 10 and configured to enable the flow F of the extraction solution from the solubilization chamber 18 to the sampling chamber 19.

According to an alternative or additional embodiment, not illustrated, the passage means is at least partially fashioned in the walls of the container defining the extraction portion and/or sampling portion.

In order to withdraw the filtered extraction solution 3a from the sampling chamber 19, the sampling chamber 19 has, for example, an aperture 20 communicating with the outside. Preferably, there is provided a cover 21 suitable for enabling access to the sampling chamber 19 through the aperture 20. In particular, the aperture 20 defines a sampling point 22 closed off by means of at least one pierceable layer defining the cover 21. According to the illustrated example, the sampling point is provided in the closure portion 8 comprising the sampling chamber 19.Said sampling point 22 communicates, or terminates, at one end with the sampling chamber 19 and at the opposite end with the outside of the closure portion 8.

In other words, the sampling point 22 is directly accessible from the outside of the closure portion 8.

Said sampling point 22 is defined by a cylindrical body. Said cylindrical body is located inside the closure portion 8. Preferably, said sampling point 22 is defined by a cylindrical body contained inside the seat fashioned in the closure portion 8.

Said sampling point 22 is entirely contained inside the seat without comprising any portions projecting outside the closure portion.

In other words, said sampling point 22 is defined by a cylindrical body contained inside the through hole formed in the closure portion.

Said sampling point 22 is entirely contained inside the through hole without comprising any portions projecting outside the closure portion 8.

During use, a container 1 corresponding to the one illustrated in figures 1-6, is used starting from an extraction portion 2 comprising the extraction solution 3 and preferably closed off by means of the penetrable membrane 7. The shaped wand 10 is used to withdraw the specimen and inserted into the extraction portion 2 so that the shaped wand 10 is disposed inside the extraction solution 3 and inside the solubilization chamber 18. In other words, the solubilization chamber 18 contains the extraction solution 3.

The closure portion 8 is fixed, for example by screwing, on the extraction portion 2, thus isolating the environment inside the container.

Subsequently, the container is turned and brought, for example, into the configuration of figure 6, so that the extraction solution 3 drops as a result of the force of weight from the solubilization chamber 18 into the sampling chamber 19, passing through the filtering element 17, which holds back the particles of the specimen which did not completely dissolve in the extraction solution.

The flow F is assured through the shaped wand 10 by means of the openings 15a.

One can thus access the filtered extraction solution 3a contained in the sampling chamber 19, for example, by piercing the pierceable layer of the sampling point 22 with a needle 23.

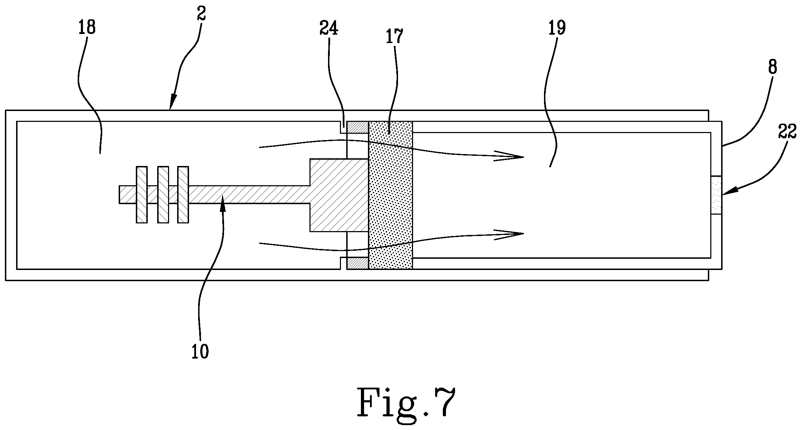

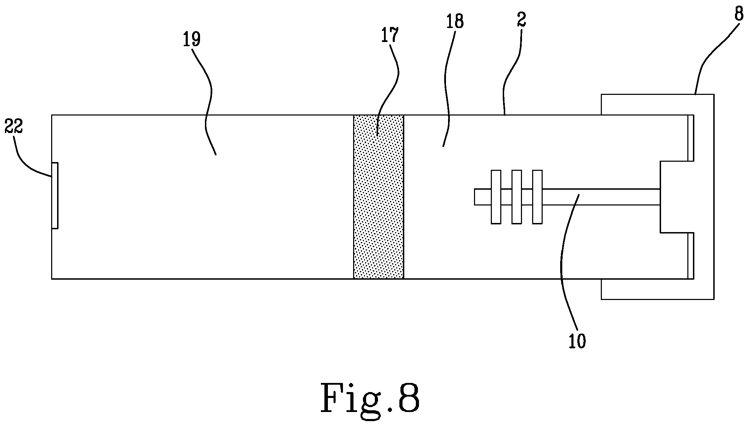

Figures 7 and 8 illustrate two possible alternatives of the container 1, wherein the elements in common with the embodiment illustrated in figures 1-6 have been indicated with the same numerical references.

Figure 7 differs from figure 2 in that both the solubilization chamber 18 and the sampling chamber 19 are defined inside the extraction portion 2. The shaped wand 10 abuts against reliefs 24 of the extraction portion 2. The closure portion 8 abuts against the filtering element 17 and keeps it in position against the shaped wand 10 and the reliefs 24.

Figure 8 differs from figure 7 in that the filtering element 17 is centrally disposed in the extraction portion 2; thus the shaped wand 10 is not interposed between the solubilization chamber 18 and the sampling chamber 19.

## Claims

1. A container (1) for collecting faecal specimens comprising:
an extraction portion (2) having a solubilization chamber (18) suitable for containing an extraction solution (3);
a closure portion (8) for closing said container (1) having a sampling chamber (19);
a connection means (9) interposed between said extraction portion (2) and said closure portion (8) and configured to connect said extraction portion (2) and said closure portion (8) to define a closed configuration of said container (1) in which said sampling chamber (19) and said solubilization chamber (18) are arranged to opposite portions of the container (1);
a shaped wand (10) is associated to the closure portion (8) and suitable for collecting the specimens, said shaped wand (10) being suitable for being axially inserted into said extraction portion (2) in said closed configuration,
at least one filtering element (17) which, at least in said closed configuration of the container (1), divides said solubilization chamber (18) from said sampling chamber (19), said filtering element (17) being suitable for enabling a flow (F) of the extraction solution from the solubilization chamber (18) to the sampling chamber (19);
**characterized in that** said extraction portion (2) comprises a penetrable membrane (7) disposed so as to close off the extraction portion (2) in an open configuration of the container (1) in which said extraction portion (2) and closure portion (8) are decoupled from each other, and wherein said shaped wand (10) comprises a tip (12) configured to tear said breakable membrane (7) and is associated with the closure portion (8) in such a way as to achieve said tearing of the penetrable membrane (7) during the passage from the open configuration to the closed configuration of the container (1).

2. A container (1) for collecting faecal specimens according to claim 1, wherein, in said closed configuration, said shaped wand (10) is suitable for being axially inserted into said solubilization chamber (18) and said sampling chamber (19) is disposed on the opposite side of the solubilization chamber (18) relative to the shaped wand (10).

3. A container (1) for collecting faecal specimens according to claim 1 or 2, comprising passage means configured to enable the flow of the extraction solution (3) from the solubilization chamber (18) to the sampling chamber (19).

4. A container (1) for collecting faecal specimens according to claim 3, wherein said passage means comprises at least one opening (15a) fashioned in said shaped wand (10) and configured to enable the flow (F) of the extraction solution (3) from the solubilization chamber (18) to the sampling chamber (19).

5. A container (1) for collecting faecal specimens according to one or more of the preceding claims, wherein said shaped wand (10) comprises a plurality of projections (15) extending in a radial direction, wherein the space between two consecutive projections defines an opening (15a) configured to enable the flow (F) of the extraction solution (3) from the solubilization chamber (18) to the sampling chamber (19).

6. A container (1) for collecting faecal specimens according to claim 5, wherein said shaped wand (10) comprises at least one perimeter ring (13) for joining said projections (15), said perimeter ring (13) being suitable for abutting against a free edge (6) of said extraction portion (2).

7. A container (1) for collecting faecal specimens according to claim 5 or 6, wherein said projections (15) extend axially along a discrete portion of the shaped wand (10), preferably equal to 1/3 of the axial length of the shaped wand (10), thus forming fins (16).

8. A container (1) for collecting faecal specimens according to one or more of the preceding claims, wherein said filtering element (17) is associated with said closure portion (8).

9. A container (1) for collecting faecal specimens according to one or more of the preceding claims, wherein said filtering element (17) is associated with said shaped wand (10).

10. A container (1) for collecting faecal specimens according to one or more of the preceding claims, wherein said filtering element (17) is packed between said shaped wand (10) and said closure portion (8).

11. A container (1) for collecting faecal specimens according to one or more of the preceding claims, wherein said sampling chamber (19) comprises at least one sampling point (22) closed off by means of at least one pierceable layer.

12. A container (1) for collecting faecal specimens according to one or more of the preceding claims, wherein said container (1) has an at least partially tubular conformation along an axis of extension (4) and wherein, at least in said closed configuration, said sampling chamber (19) and said solubilization chamber (18) are disposed in opposite portions of the container (1) along said an axis of extension (4).

13. A container (1) for collecting faecal specimens according to one or more of the preceding claims, comprising a supporting base (5) configured to be associated with a machine for treating the extraction solution (3), for example a centrifuge, said supporting base (5) being preferably associated with said extraction portion (2).

14. A container (1) for collecting faecal specimens according to claim 12, wherein said supporting base (5) has a cup-like conformation.

15. A container (1) for collecting faecal specimens according to one or more of the preceding claims, wherein said filtering element (17) comprises a polypropylene membrane.

## Patentansprüche

1. Behälter (1) zur Entnahme von Fäkalproben, umfassend:
einen Extraktionsabschnitt (2) mit einer Solubilisierungskammer (18), die zum Enthalten einer Extraktionslösung (3) geeignet ist;
einen Verschlussabschnitt (8) zum Verschließen des Behälters (1) mit einer Probenahmekammer (19);
ein Verbindungsmittel (9), das zwischen dem Extraktionsabschnitt (2) und dem Verschlussabschnitt (8) angeordnet und konfiguriert ist, um den Extraktionsabschnitt (2) und den Verschlussabschnitt (8) zu verbinden, um eine geschlossene Konfiguration des Behälters (1) zu definieren, wobei die Probenahmekammer (19) und die Solubilisierungskammer (18) an gegenüberliegenden Abschnitten des Behälters (1) angeordnet sind;
ein geformter Stab (10) ist mit dem Verschlussabschnitt (8) assoziiert und zur Entnahme der Proben geeignet ist, wobei der geformte Stab (10) zum axialen Einführen in den Extraktionsabschnitt (2) in der geschlossenen Konfiguration geeignet ist,
zumindest ein Filterelement (17), das zumindest in der geschlossenen Konfiguration des Behälters (1) die Solubilisierungskammer (18) von der Probenahmekammer (19) trennt, wobei das Filterelement (17) zur Ermöglichung eines Flusses (F) der Extraktionslösung von der Solubilisierungskammer (18) zur Probenahmekammer (19) geeignet ist;
**dadurch gekennzeichnet, dass** der Extraktionsabschnitt (2) eine durchdringbare Membran (7) umfasst, die so angeordnet ist, dass sie den Extraktionsabschnitt (2) in einer offenen Konfiguration des Behälters (1) absperrt, in der der Extraktionsabschnitt (2) und der Verschlussabschnitt (8) voneinander entkoppelt sind und wobei der geformte Stab (10) eine Spitze (12) umfasst, die zum Aufreißen der zerbrechlichen Membran (7) konfiguriert ist und mit dem Verschlussabschnitt (8) assoziiert ist, so dass das Aufreißen der durchdringbaren Membran (7) während des Übergangs von der offenen Konfiguration zur geschlossenen Konfiguration des Behälters (1) erzielt wird.

2. Behälter (1) zur Entnahme von Fäkalproben nach Anspruch 1, wobei in der geschlossenen Konfiguration der geformte Stab (10) zum axialen Einführen in die Solubilisierungskammer (18) geeignet ist und die Probenahmekammer (19) auf der gegenüberliegenden Seite der Solubilisierungskammer (18) relativ zum geformten Stab (10) angeordnet ist.

3. Behälter (1) zur Entnahme von Fäkalproben nach Anspruch 1 oder 2, umfassend Durchgangsmittel, die konfiguriert sind, um den Fluss der Extraktionslösung (3) von der Solubilisierungskammer (18) zur Probenahmekammer (19) zu ermöglichen.

4. Behälter (1) zur Entnahme von Fäkalproben nach Anspruch 3, wobei die Durchgangsmittel zumindest eine Öffnung (15a) umfassen, die in dem geformten Stab (10) ausgebildet ist und konfiguriert ist, um den Fluss (F) der Extraktionslösung (3) von der Solubilisierungskammer (18) zur Probenahmekammer (19) zu ermöglichen.

5. Behälter (1) zur Entnahme von Fäkalproben nach einem oder mehreren der vorhergehenden Ansprüche, wobei der geformte Stab (10) eine Vielzahl an Vorsprüngen (15) umfasst, die sich in radialer Richtung erstrecken, wobei der Raum zwischen zwei aufeinanderfolgenden Vorsprüngen eine Öffnung (15a) definiert, die konfiguriert ist, um den Fluss (F) der Extraktionslösung (3) von der Solubilisierungskammer (18) zur Probenahmekammer (19) zu ermöglichen.

6. Behälter (1) zur Entnahme von Fäkalproben nach Anspruch 5, wobei der geformte Stab (10) zumindest einen Umfangsring (13) zum Zusammenfügen der Vorsprünge (15) umfasst, wobei der Umfangsring (13) zum Anstoßen gegen einen freien Rand (6) des Extraktionsabschnitts (2) geeignet ist.

7. Behälter (1) zur Entnahme von Fäkalproben nach Anspruch 5 oder 6, wobei sich die Vorsprünge (15) axial entlang eines separaten Abschnitts des geformten Stabes (10) erstrecken, vorzugsweise gleich 1/3 der axialen Länge des geformten Stabes (10), wodurch Rippen (16) gebildet werden.

8. Behälter (1) zur Entnahme von Fäkalproben nach einem oder mehreren der vorhergehenden Ansprüche, wobei das Filterelement (17) mit dem Verschlussabschnitt (8) assoziiert ist.

9. Behälter (1) zur Entnahme von Fäkalproben nach einem oder mehreren der vorhergehenden Ansprüche, wobei das Filterelement (17) mit dem geformten Stab (10) assoziiert ist.

10. Behälter (1) zur Entnahme von Fäkalproben nach einem oder mehreren der vorhergehenden Ansprüche, wobei das Filterelement (17) zwischen dem geformten Stab (10) und dem Verschlussabschnitt (8) gepackt ist.

11. Behälter (1) zur Entnahme von Fäkalproben nach einem oder mehreren der vorhergehenden Ansprüche, wobei die Probenahmekammer (19) zumindest einen Probenahmepunkt (22) umfasst, der mittels zumindest einer durchstechbaren Schicht abgesperrt ist.

12. Behälter (1) zur Entnahme von Fäkalproben nach einem oder mehreren der vorhergehenden Ansprüche, wobei der Behälter (1) eine zumindest teilweise röhrenförmige Ausbildung entlang einer Erstreckungsachse (4) aufweist und wobei zumindest in der geschlossenen Konfiguration die Probenahmekammer (19) und die Solubilisierungskammer (18) in gegenüberliegenden Abschnitten des Behälters (1) entlang der Erstreckungsachse (4) angeordnet sind.

13. Behälter (1) zur Entnahme von Fäkalproben nach einem oder mehreren der vorhergehenden Ansprüche, umfassend eine Stützbasis (5), die konfiguriert ist, um mit einer Maschine zum Behandeln der Extraktionslösung (3), beispielsweise einer Zentrifuge, assoziiert zu werden, wobei die Stützbasis (5) vorzugsweise mit dem Extraktionsabschnitt (2) assoziiert ist.

14. Behälter (1) zur Entnahme von Fäkalproben nach Anspruch 12, wobei die Stützbasis (5) eine becherartige Ausbildung aufweist.

15. Behälter (1) zur Entnahme von Fäkalproben nach einem oder mehreren der vorhergehenden Ansprüche, wobei das Filterelement (17) eine Polypropylenmembran umfasst.

## Revendications

1. Récipient (1) de collecte d'échantillons de matières fécales comprenant :
une partie d'extraction (2) comportant une chambre de solubilisation (18) adaptée pour contenir une solution d'extraction (3) ;
une partie de fermeture (8) servant à fermer ledit récipient (1) comportant une chambre d'échantillonnage (19) ;
des moyens de raccordement (9) interposés entre ladite partie d'extraction (2) et ladite partie de fermeture (8) et configurés pour raccorder ladite partie d'extraction (2) et ladite partie de fermeture (8) pour définir une configuration de fermeture dudit récipient (1) dans lequel ladite chambre d'échantillonnage (19) et ladite chambre de solubilisation (18) sont disposées aux parties opposées du récipient (1) ;
une baguette façonnée (10) est associée à la partie de fermeture (8) et étant adaptée pour recueillir les échantillons, ladite baguette façonnée (10) étant adaptée pour être introduite axialement dans ladite partie d'extraction (2) dans ladite configuration de fermeture,
au moins un élément de filtrage (17) qui, au moins dans ladite configuration de fermeture du récipient (1), divise ladite chambre de solubilisation (18) de ladite chambre d'échantillonnage (19), ledit élément de filtrage (17) étant adapté pour permettre un écoulement (F) de la solution d'extraction de la chambre de solubilisation (18) vers la chambre d'échantillonnage (19) ;
**caractérisé en ce que** ladite partie d'extraction (2) comprend une membrane pénétrable (7) disposée de manière à fermer la partie d'extraction (2) dans une configuration d'ouverture du récipient (1) dans laquelle ladite partie d'extraction (2) et la partie de fermeture (8) sont découplées l'une de l'autre, et dans lequel ladite baguette façonnée (10) comprend une pointe (12) configurée pour déchirer ladite membrane cassable (7) et est associée à la partie de fermeture (8) de manière à réaliser ladite déchirure de la membrane pénétrable (7) pendant le passage de la configuration d'ouverture à la configuration de fermeture du récipient (1).

2. Récipient (1) de collecte d'échantillons de matières fécales selon la revendication 1, dans lequel, dans ladite configuration de fermeture, ladite baguette façonnée (10) est adaptée pour être introduite axialement dans ladite chambre de solubilisation (18) et ladite chambre d'échantillonnage (19) est disposée sur le côté opposé de la chambre de solubilisation (18) par rapport à la baguette façonnée (10).

3. Récipient (1) de collecte d'échantillons de matières fécales selon la revendication 1 ou 2, comprenant des moyens de passage configurés pour permettre l'écoulement de la solution d'extraction (3) de la chambre de solubilisation (18) à la chambre d'échantillonnage (19).

4. Récipient (1) de collecte d'échantillons de matières fécales selon la revendication 3, dans lequel lesdits moyens de passage comprennent au moins une ouverture (15a) façonnée dans ladite baguette façonnée (10) et configurés pour permettre l'écoulement (F) de la solution d'extraction (3) de la chambre de solubilisation (18) à la chambre d'échantillonnage (19).

5. Récipient (1) de collecte d'échantillons de matières fécales selon une ou plusieurs des revendications précédentes, dans lequel ladite baguette façonnée (10) comprend une pluralité de saillies (15) se prolongeant dans une direction radiale, dans lequel l'espace entre deux saillies consécutives définit une ouverture (15a) configurée pour permettre l'écoulement (F) de la solution d'extraction (3) de la chambre de solubilisation (18) à la chambre d'échantillonnage (19).

6. Récipient (1) de collecte d'échantillons de matières fécales selon la revendication 5, dans lequel ladite baguette façonnée (10) comprend au moins un anneau périphérique (13) servant à joindre lesdites saillies (15), ledit anneau périphérique (13) étant adapté pour se mettre en butée contre un bord libre (6) de ladite partie d'extraction (2).

7. Récipient (1) de collecte d'échantillons de matières fécales selon la revendication 5 ou 6, dans lequel lesdites saillies (15) se prolongent axialement le long d'une portion distincte de la baguette façonnée (10), de préférence égale à 1/3 de la longueur axiale de la baguette façonnée (10), formant ainsi des ailettes (16).

8. Récipient (1) de collecte d'échantillons de matières fécales selon une ou plusieurs des revendications précédentes, dans lequel ledit élément de filtrage (17) est associé à ladite partie de fermeture (8).

9. Récipient (1) de collecte d'échantillons de matières fécales selon une ou plusieurs des revendications précédentes, dans lequel ledit élément de filtrage (17) est associé à ladite baguette façonnée (10).

10. Récipient (1) de collecte d'échantillons de matières fécales selon une ou plusieurs des revendications précédentes, dans lequel ledit élément de filtrage (17) est placé entre ladite baguette façonnée (10) et ladite partie de fermeture (8).

11. Récipient (1) de collecte d'échantillons de matières fécales selon une ou plusieurs des revendications précédentes, dans lequel ladite chambre d'échantillonnage (19) comprend au moins un point d'échantillonnage (22) fermé au moyen d'au moins une couche perçable.

12. Récipient (1) de collecte d'échantillons de matières fécales selon une ou plusieurs des revendications précédentes, dans lequel ledit récipient (1) a une conformation au moins partiellement tubulaire le long d'un axe d'extension (4) et dans lequel, au moins dans ladite configuration de fermeture, ladite chambre d'échantillonnage (19) et ladite chambre de solubilisation (18) sont disposées dans des parties opposées du récipient (1) le long dudit axe d'extension (4).

13. Récipient (1) de collecte d'échantillons de matières fécales selon une ou plusieurs des revendications précédentes, comprenant une base de support (5) configurée pour être associée à une machine servant à traiter la solution d'extraction (3), par exemple une centrifugeuse, ladite base de support (5) étant de préférence associée à ladite partie d'extraction (2).

14. Récipient (1) de collecte d'échantillons de matières fécales selon la revendication 12, dans lequel ladite base de support (5) a une conformation en forme de gobelet.

15. Récipient (1) de collecte d'échantillons de matières fécales selon une ou plusieurs des revendications précédentes, dans lequel ledit élément de filtrage (17) comprend une membrane en polypropylène.
